# EUROPEAN PATENT APPLICATION

(11) **EP 4 477 755 A1**
(43) Date of publication of application: **18.12.2024**
(21) Application number: 23382585.0
(22) Date of filing: 13.06.2023
(51) Int. Cl.: C12P 7/40, C12M 1/04, C12M 1/00

(54) **BIOPROCESS FOR THE PRODUCTION OF ELONGATED CARBOXYLIC ACIDS**

(71) Applicant: Universitat de Girona, 17071 Girona (ES)
(72) Inventor: DESSÌ, Paolo, 17004 GIRONA (ES); PUIG BROCH, Sebastià, 17455 CALDES DE MALAVELLA (ES); ROMANS CASAS, Meritxell, 17472 L'ARMENTERA (ES); BALAGUER CONDOM, Maria Dolors, 17003 GIRONA (ES)
(74) Representative: ZBM Patents - Zea, Barlocci & Markvardsen

(57) **Abstract**

The present disclosure refers to a process for the production of C4-C10 carboxylic acid by carbon chain elongation, comprising the steps of: (i) providing a hydrophobic membrane (1) with a first side (2) and a second side (3) opposite to said first side (2); (ii) bringing a feed stream (4) comprising organic C1-C4 compounds, of which at least one is an electron donor, into contact with said first side (2) of said hydrophobic membrane (1); (iii) bringing an aqueous solution (5) comprising enzymes capable of carrying out carbon chain elongating of organic C1-C4 compounds into contact with said second side (3) of said hydrophobic membrane (1), thereby causing the organic C1-C4 compounds concentration-driven diffusion from said feed stream (4) through said hydrophobic membrane (1) to said aqueous solution (5); (iv) subjecting the organic C1-C4 compounds diffused into the aqueous solution (5) to enzymatic carbon chain elongation in the presence of the electron donor; and (v) separating the formed C4-C10 carboxylic acid from the aqueous solution (5).

## Description

### Technical Field

The present disclosure pertains to the field of waste treatment, in particular to valorisation of organic wastes as well as other carbon-containing waste through enzymatic carbon chain elongation.

### Background Art

Global warming and dwindling fossil energy resources have boosted the search for renewable energies. On the other side, traditional methods of producing chemicals from fossil reserves result in environmental concerns, prompting researchers to explore more eco-friendly approaches, such as utilizing organic waste or industrial by-products to obtain these chemicals.

Biogas generation through anaerobic digestion from waste has advanced technologically but the release of methane, a potent greenhouse gas, remains a concern. Bioethanol produced from biomass poses problems related to availability of raw materials, as it depends on the starch and sugar content of the biomass employed. Even though considered as renewable, big environmental concerns are arising due to water requirements for crop growth and the destruction of forestry to clear cultivable land, not to forget that the significant increase of crops dedicated to produce biofuel has sometimes caused an increase in the price of edible corn and other foods.

As an alternative, organic wastes and by-products can be used as a source for obtaining biofuel and/or valuable chemicals (green chemicals). The conversion of said renewable resources into short chain carboxylic acids (SCCA), including acetic (C2), propionic (C3), and butyric (C4) acids, through microbial fermentation is a viable technology. In fermentation, bacteria break down biomass materials into simple chemicals, such as ethanol, SCCA, CO₂, and CH₄. Fermentation has the potential to play a crucial role in both waste treatment and the generation of value-added chemicals like ethanol and SCCA. Furthermore, the biological conversion of ethanol and SCCA into medium chain carboxylic acids (MCCA), such as hexanoic, heptanoic, and octanoic acids, has shown great promise. MCCAs can be produced from SCCAs elongation via reverse β-oxidation pathway in the presence of electron donor (e.g., ethanol) (EP2271764). *Clostridium kluyveri* is the most studied genus that can conduct this reaction with ethanol as the electron donor. MCCAs are regarded as precursors for various valuable chemical products including fragrances, pharmaceuticals, lubricants, plastics, antimicrobials, feed additives and biofuels.

Different types of waste materials have been employed for MCCA production through microbial elongation, such as, for example, waste activated sludge (He et al, 2021) and kitchen waste (Huo et al, 2022). However, the available technology suffers from several drawbacks: 1) Substrates containing high concentrations of ethanol and acetic acid (e.g. above 100-200 mM) cannot be conveniently used for microbial elongation, as they are toxic for bacteria. 2) Many wastewaters and fermentates contain ethanol and SCCA at sub-optimal ratio for chain elongation. In many cases, acetic acid is present in higher concentration than ethanol and this negatively influences product selectivity. 3) Open cultures are often used for scaled-up process but may result in the formation of undesired products. 4) One-step processes are sub-optimal due to the different optimal conditions for the fermentative and chain elongating microbial community, whereas a separate fermentation step may suffer of product inhibition and requires substantial expenses, for example, related to pH adjustment, feed product transportation, etc.

There is a need in the art to provide improved technology for producing valuable MCCA.

### Summary of Invention

The present inventors have developed an innovative solution for optimizing the production of elongated carboxylic acids from wastes or fermentates (including broths deriving from solid, liquid and gas fermentation processes) that contain C1-C4 carboxylic acids and ethanol at different proportions. The inventive solution comprises physical separation of the feed stream comprising waste or fermentate from an aqueous solution containing enzymes capable of carrying out carbon chain elongation through a hydrophobic membrane. The ethanol and C1-C4 carboxylic acids comprised in the waste or fermentate selectively diffuse from the feed stream through the hydrophobic membrane to meet the enzymatic solution, with the subsequent production of elongated carboxylic acids by enzymatic carbon chain elongation.

A first aspect of the disclosure thus provides a process for the production of C4 -C10 carboxylic acids by carbon chain elongation, comprising the steps of:
(i) providing a hydrophobic membrane with a first side and a second side opposite to said first side;
(ii) bringing a feed stream comprising organic C1-C4 compounds, of which at least one is an electron donor, into contact with said first side of said hydrophobic membrane,
(iii) bringing an aqueous solution comprising enzymes capable of carrying out carbon chain elongating of organic C1-C4 compounds into contact with said second side of said hydrophobic membrane, thereby causing the organic C1-C4 compounds concentration-driven diffusion from said feed stream through said hydrophobic membrane to said aqueous solution;
(iii) subjecting the organic C1-C4 compounds diffused into the aqueous solution comprising enzymes capable of carrying out carbon chain elongation to obtain enzymatic carbon chain elongation in the presence of the electron donor; and
(iv) separating the formed C4 -C10 carboxylic acid from the aqueous solution comprising enzymes capable of carrying out carbon chain elongating.

Some feed streams of wastewaters and fermented wastes or by-products contain high concentrations of volatile organic compounds such as SCCA and ethanol but also contain many other compounds (microorganisms, sugars, contaminants such as metals, etc) that can reduce the yield of the elongating process. The hydrophobic membrane acts as a barrier for microorganisms and soluble compounds (e.g., sugars, nutrients) but allows the concentration-driven diffusion of volatile organic compounds such as ethanol and undissociated SCCA. The diffused ethanol and SCCA are used to produce the elongated compounds via the reverse β-oxidation pathway. This will keep the ethanol and SCCA concentration low in the second side of the membrane, further favouring their diffusion from the feed stream. Most importantly, ethanol diffuses through the hydrophobic membrane at a higher rate than acetic acid and, depending on the conditions, may also diffuse at a higher rate than other SCCAs. Thus, higher ethanol:SCCA ratios may be obtained in the second side, which are beneficial for chain elongation. Furthermore, if the solution on the second side is kept at a pH > 6.5, the product will be mostly in the dissociated form (i.e., butyrate, valerate, hexanoate, heptanoate, octanoate), and will not diffuse back towards the feed stream, thus accumulating in the second side (as only undissociated carboxylic acids can cross the hydrophobic membrane).

The present process has several advantages:
1) The hydrophobic membrane allows concentration-driven diffusion of electron donor (usually ethanol) and SCCAs via liquid-liquid pertraction without the use of any toxic solvent and without any energy requirement.
2) The process allows to obtain a favourable ratio for elongation between the SCCA substrates and the electron donor (usually ethanol), regardless of the ratio in the feed stream and at very low cost.
3) Because the feed stream is physically separated from the elongating reaction, the process allows for the use of pure enzymes, enzymatic systems or microbial cultures containing microorganisms capable of carrying out the carbon chain elongation. Furthermore, specific conditions (pH, co-factors, growth media) can be used for the elongating step without risk of contamination.
4) Waste fermentation and elongation can be simultaneously performed while keeping optimal conditions for both, since both processes are physically separated. Again, physical separation of the elongating process allows for optimisation of the conditions for chain elongation, independent of the conditions required for waste fermentation, which may also be optimised as required. The continuous flow of alcohols and SCCA from the feed stream to the chain elongating solution also alleviates product inhibition and reduces expenses for pH control.
5) The dosing of ethanol and substrate SCCAs can be easily fine-tuned based on the requirements of the process.

The produced C4-C10 carboxylic acids as such may be used as substrates to produce biofuels by a subsequent (bio)chemical reaction such as by ketonic decarboxylation, hydrogenation or solventogenesis.

Accordingly, a second aspect of the disclosure refers to a process for producing biofuel comprising the step of producing C4-C10 carboxylic acids by a process as defined in the first aspect.

A third aspect of the disclosure refers to a bioreactor, said bioreactor comprising:
(i) a bioreactor chamber filled with an aqueous solution comprising enzymes capable of carrying out chain elongation of organic C1-C4 compounds,
(ii) a polymeric hydrophobic membrane in the form of a tubular element through the inside of which a feed stream comprising organic C1 -C4 compounds can flow, wherein said membrane is submerged in the aqueous solution comprising enzymes capable of carrying out chain elongation, and (iii) a recirculation system for the feed stream.

### Brief Description of Drawings

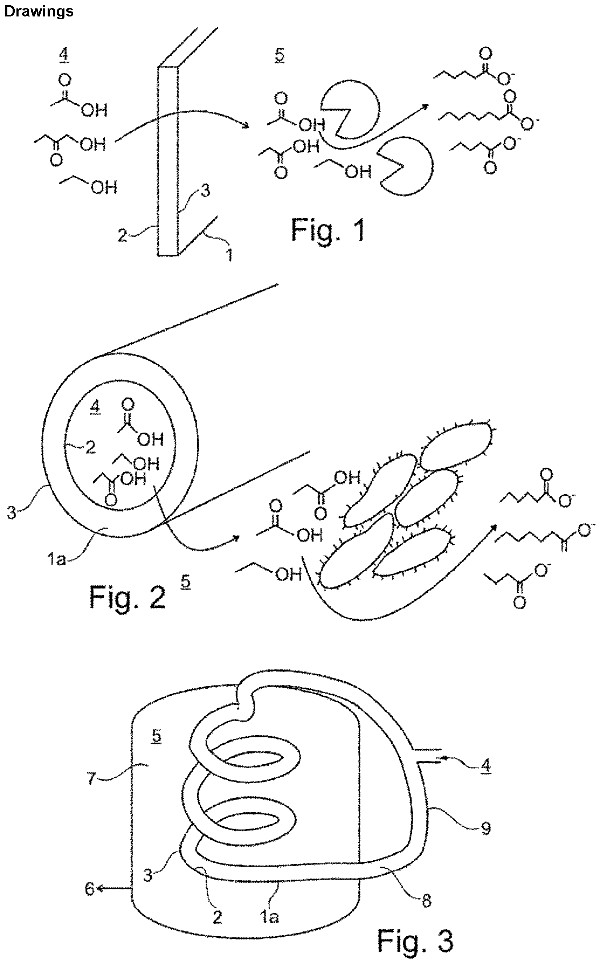

Figure 1. Schematic representation of a process according to the first aspect of the disclosure. (1) Hydrophobic membrane. (2) First side of the hydrophobic membrane. (3) Second side of the hydrophobic membrane. (4) Feed stream comprising organic C1-C4 compounds. (5) aqueous solution comprising enzymes capable of carrying out carbon chain elongation of organic C1-C4 compounds.
Figure 2. Schematic representation of an embodiment according to the first aspect of the disclosure wherein the hydrophobic membrane is a tubular element, and the elongation is carried out by microorganisms. (1a) Hydrophobic membrane in the form of a tubular element. (2) First side of the hydrophobic membrane. (3) Second side of the hydrophobic membrane. (4) Feed stream comprising organic C1-C4 compounds. (5a) Aqueous microbial culture comprising microorganisms capable of carrying out carbon chain elongation of organic C1-C4 compounds.
Figure 3. Schematic representation of a bioreactor (6) according to the third aspect of the disclosure (SIL-HEX). (1a) Hydrophobic membrane in the form of a tubular element. (4) Feed stream comprising organic C1-C4 compounds. (5a) Aqueous microbial culture comprising microorganisms capable of carrying out carbon chain elongation of organic C1-C4 compounds. (7) Bioreactor chamber. (8) Lumen of the hydrophobic membrane in the form of a tubular element. (9) Recirculation system.

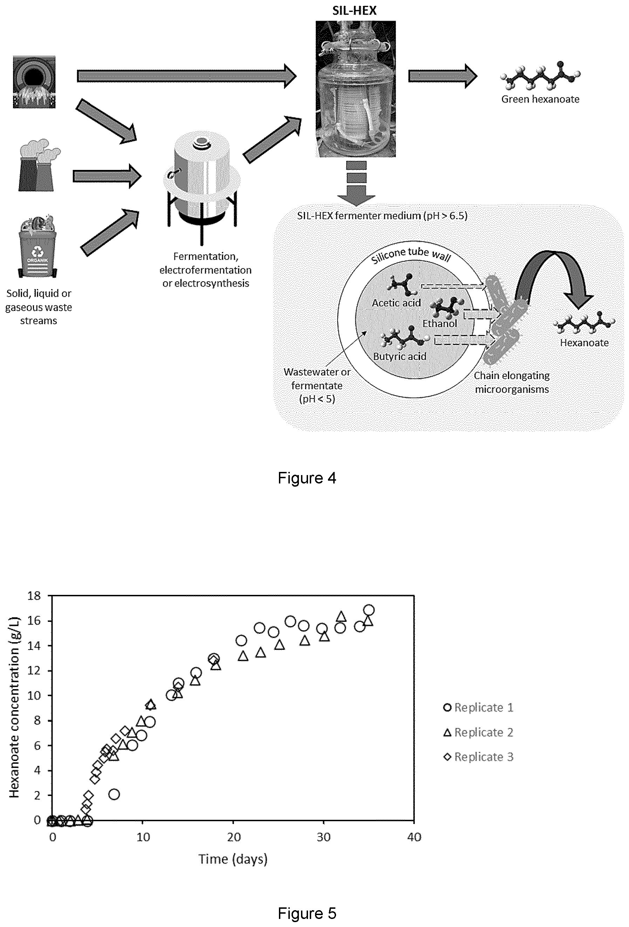

Figure 4. Schematic representation of a particular embodiment of the disclosure. Solid waste, wastewaters, industrial by-products and CO₂ containing gas streams can be converted into a mixture of carboxylic acids (mainly acetic and butyric acids) and alcohols (mainly ethanol) by processes such as fermentation, electrofermentation or electrosynthesis. The fermentate will then be recirculated in the SIL-HEX bioreactor. Wastewaters containing high (>100 mM) concentrations of ethanol and short chain carboxylic acids (preferably in their undissociated form) can be fed directly to SIL-HEX. The molecules will then diffuse through a silicone tubing at a rate proportional to their hydrophobicity and /or volatility. Hence, both ethanol and butyric acid will diffuse faster than acetic acid. Chain elongating microorganisms will then produce hexanoate using acetic/butyric acid as substrate and ethanol as electron donor.
Figure 5. Hexanoate concentration profile obtained in the Sil-HEX fermenter under the conditions described in the Example. The experiment was conducted in three independent replicates.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The present disclosure is directed to a method for the production of C4-C10 carboxylic acids by carbon chain elongation from feed streams containing organic C1-C4 compounds, such as SCCA and ethanol, using a hydrophobic membrane and conditions appropriate for the selective concentration-driven diffusion of said organic C1-C4 compounds to meet a solution comprising enzymes capable of carrying out carbon chain elongation of said organic C1-C4 compounds.

As used herein, the indefinite articles "a" and "an" are synonymous with "at least one" or "one or more." Unless indicated otherwise, definite articles used herein, such as "the" also include the plural of the noun.

The term "short chain carboxylic acid" or "SCCA" is generally understood as C1 to C5 carboxylic acids. The term "medium chain carboxylic acid" or "MCCA" generally refers to C6 to C12 carboxylic acids. As would be readily acknowledged by the skilled person, the borders between SCCA and MCCA is not clear-cut.

The term "hydrophobic membrane" refers to membranes that do not favour reactions with aqueous media.

"Aqueous" is understood to define a system that involves water. The term "aqueous solution" is herein understood to describe a solution or mixture in which water is the solvent, albeit not necessarily the sole solvent.

The process of the first aspect comprises: bringing a feed stream 4 comprising organic C1-C4 compounds, wherein said organic C1-C4 compounds at least comprise an electron donor, into contact with the first side 2 of a hydrophobic membrane 1; bringing an aqueous solution 5 comprising enzymes capable of carrying out carbon chain elongation of organic C1-C4 compounds into contact with the second side 3 of said hydrophobic membrane 1, thereby causing the organic C1-C4 compounds concentration-driven diffusion from said feed stream 4 through said hydrophobic membrane 1 to said aqueous solution 5; subjecting the organic C1-C4 compounds diffused into the aqueous solution 5 to enzymatic carbon chain elongation in the presence of the electron donor; and separating the formed C4 -C10 carboxylic acid from the aqueous solution 5.

A scheme of the proposed process for the production of elongated carboxylic acids according to the first aspect is provided in Figures 1 and 2.

In one embodiment, the hydrophobic membrane 1 comprises or consists of a dense hydrophobic polymer. By "dense" it is understood that the membrane has a structure showing no visible pores in the range of electron microscopy. In particular embodiments, the hydrophobic membrane comprises or consists of a polymer selected from the group consisting of a polysiloxane, polytetrafluotoethylene, and polypropylene. In particular embodiments the polysiloxane is polydimethylsiloxane (silicone). In a more particular embodiment the hydrophobic membrane comprises or consists of polydimethylsiloxane (silicone).

In one embodiment the hydrophobic membrane is a sheet or a film. In a particular embodiment, the hydrophobic membrane 1 is a tubular element 1a (as shown in figure 2). In another particular embodiment, the tubular membrane is a hollow fibre, in particular a plurality of hollow fibres (also called "capillaries"). In a preferred configuration, the first side 2 of the membrane 1 contacting the feed material is the inner surface (or lumen) of the tubular element. In a particular configuration of the process of the first aspect, the step (ii) of bringing said feed stream 4 into contact with the first side 2 of the hydrophobic membrane 1 is carried out by flowing the feed stream 4 through the first side 2 of the hydrophobic membrane 1. In a particular embodiment, when the membrane is a tubular element 1a, said tubular element is coiled. In a more particular embodiment, the feed stream 4 flows through the first side 2 of the hydrophobic tubular membrane at a flow velocity in the range from 1 to 5000 m/h, in particular from 50 to 1000 m/h, more in particular from 100 to 500 m/h. In a very particular embodiment of the process of the first aspect, the feed stream 4 is recirculated after flowing through the first side 2 of the hydrophobic membrane 1.

In one embodiment, the wall thickness of the membrane 1 or, otherwise worded, the width of the membrane 1, may be from 0.01 to 4 mm, in particular from 0.01 to 2 mm, more in particular from 0.25 to 1 mm, for example 0.3 mm, 0.4 mm, 0.5 mm, 0.6 mm, 0.7 mm, 0.8 mm or 0.9 mm. The membrane thickness determines the diffusion pathway that the organic C1-C4 compounds have to travel to reach the aqueous solution 5 comprising enzymes capable of carrying out carbon chain elongation. The membrane thickness may also affect the selectivity of the diffusion. For example, increasing the membrane thickness increases the ethanol:acetic acid ratio. When the membrane 1 is a tubular element 1a, said tubular element 1a may have an internal diameter of 0.5 mm to 5 cm, in particular from 0.5 mm to 1 cm, more in particular from 1 to 3 mm, for example 2 mm. When the membrane is a hollow fibre or plurality of hollow fibres, said fibres may have an internal diameter of 500 µm or less, in particular of 300 µm or less, more in particular the fibres may have an internal diameter in the range of 50-250 µm, or in the range of 20-300 µm, such as in the range of 40-250 µm, or in the range of 50-100 µm. In these cases, where the membrane 1 is a hollow fibre, the thickness of the membrane may be in the range from 10 to 500 µm, in particular from 10 to 50 µm.

As outlined above, the feed stream 4 comprises organic C1-C4 compounds. In one embodiment, the feed stream 4 comprises C1-C4 alcohols and C1-C4 carboxylic acids. In more particular embodiments, the C1-C4 alcohols comprise ethanol and/or propanol, in particular ethanol. In other more particular embodiments, the C1-C4 carboxylic acids comprise acetic acid, propionic acid, butyric acid, succinic acid, valeric acid, caproic acid, or mixtures thereof. In even more particular embodiments, the C1-C4 carboxylic acids comprise acetic acid, propionic acid, butyric acid, or mixtures thereof. In even more particular embodiments, the C1-C4 carboxylic acids comprise acetic acid. In even more particular embodiments the feed stream 4 comprises at least ethanol. In even more particular embodiments the feed stream 4 comprises ethanol and C1-C4 carboxylic acids, in particular acetic acid. In particular embodiments the carboxylic acids in the feed stream are undissociated. In one embodiment the feed stream 4 is an aqueous solution.

The concentration of the organic C1-C4 compounds in the feed stream 4 is not particularly limited, since the selective diffusion through the hydrophobic membrane 1 results in the substrates arriving at the second side of the membrane 3 at proportions appropriate for elongation. Typically, when starting from a feed stream containing equal concentration of ethanol and C1-C4 carboxylic acids, in particular acetic acid, the compounds diffuse through the hydrophobic membrane at an ethanol:carboxylic acids ratio from 3 to 20 (i.e. for example from 3:1 to 20:1), in particular at a range from 5 to 6 (i.e. for example from 5:1 to 6:1). This allows to obtain an ethanol:C1-C4 carboxylic acid ratio in the second side of the membrane 3 in the range from 2 to 20 (i.e. for example from 2:1 to 20:1), in particular in the range from 3 to 8 (i.e. for example 3:1, 4:1, 5:1, 6:1, 7:1 or 8:1), at very low cost. In particular embodiments the C1-C4 carboxylic acid is acetic acid. These ratios can be optimised regardless of the ratio in the feed process of the disclosure by fine tuning operation parameters, in particular pH of the feed solution, hydrophobic membrane thickness, temperature and flow velocity of the feed stream 4 through the first side 2 of the hydrophobic membrane 1, more in particular, by modifying the pH of the feed stream 4 and/or the thickness of the hydrophobic membrane 1.

Furthermore, the ethanol and acetic acid dosing can be easily controlled based on the requirements of the process. In a particular embodiment, the feed stream 4 comprises at least 0.01 M, in particular, at least 0.1 M, more in particular, from 0.1 to 1M of C1-C4 alcohols and C1-C4 carboxylic acids. For example, the feed stream 4 may have from 0.05 to 250 mM of each of C1-C4 alcohols and C1-C4 carboxylic acids, such as ethanol and acetic acid.

Advantageously, the present process may produce valuable MCCAs from feed streams 4 that comprise or consist in carbon-containing wastes and by-products. Hence, the present process may be seen as a process for revalorising wastes or by-products. The process may be disclosed as a process for obtaining MCCAs, in particular C4-C10 carboxylic acids, from wastes or by-products, said process comprising the steps as defined in the first aspect.

In a particular embodiment of the first aspect, the feed stream 4 comprises or consists of carbon-containing waste or by-product. In particular the feed stream 4 comprises or consists of organic waste or by-product. In a particular embodiment, the feed stream 4 is selected from wastewater, sewage, food industry residues or by-products, such as distillery/liquor making wastewater, fermented municipal organic waste and agroindustrial waste, wine lees, winery wastewater, beer factory wastewater, whey fermentate, fermented lignocellulosics, stillage, molasses, sugars, and certain streams from chemical and pharmaceutical industries. In one embodiment of the first aspect, the feed stream 4 comprises or consists of fermented biomass and/or fractions thereof.

The ethanol and carboxylic acids in the feed stream 4 can be also obtained from inorganic carbon sources, including gaseous substrates such as CO₂ and CO, for example, the feed stream 4 may comprise or consist of fermentation products obtained from CO₂ and CO from industrial flue gas. Hence, the process of the disclosure can also be applied in combination with gas fermentation, as well as thermochemical reactors, electrochemical and bioelectrochemical cells to obtain elongated carboxylic acids from CO/CO₂.

Any source of carbon-containing feed stream is suitable as feed stream 4 in the process of the first aspect, as long as it may flow through the first side 2 of the membrane 1 and contains organic C1-C4 compounds, of which at least one is an electron donor, in particular, the feed stream advantageously comprises ethanol. Biomass complying with these criteria may be used, including organic wastes and industrial by-products. However, it is advantageous to use pretreated feed stream in which the pretreatment results in a release or an improved availability of the required organic C1-C4 compounds, as illustrated in figure 4. Thus, in one embodiment, the process of the first aspect further comprises a step of pre-treating of the feed stream 4. Such pre-treatments may comprise fermentation or other enzymatic reaction and a treatment of biomass (such as wood) with super critical water. Furthermore, it is possible that the pre-treatment step comprises a mechanical, chemical, biological or physical treatment. A mechanical treatment comprises milling, grinding, pressing and the like. Physical pre-treatments comprise an exposure to heat, water, steam and the like. Chemical pre-treatments may include acid-base treatment, oxidative methods, and solvent pre-treatment. Biological pre-treatments include enzymatic hydrolysis and fermentation. Accordingly, in particular embodiments of the first aspect, the feed stream is pre-treated biomass, wherein the pre-treatment step is selected from the group consisting of physical treatment, a mechanical treatment, a chemical treatment, a biological treatment, and combinations thereof. In a very particular embodiment, the pre-treatment comprises a fermentation process and, consequently, the feed stream is fermented biomass. The fermentation process allows conversion of materials (biomass) containing lipids, proteins and carbohydrates. Organic substrates serve both as electron donors and acceptors. The principal fermentation products are SCCAs such as acetic, propionic and butyric acid, and alcohols such as ethanol and butanol.

The fermentative pre-treatment may be any fermentation available in the art, including chemical and biological fermentation. In one embodiment, the fermentation is a biological fermentation carried out by microorganisms. In some embodiments, the fermentation is electrofermentation. Fermenting the original biomass has the object to convert large organic compounds, such as proteins, carbohydrates and lipids into smaller compounds, mainly SCCAs and lower alcohols (in particular ethanol).

In some embodiments, the pre-treatment step is carried out in a separate equipment. In other embodiments, the pre-treatment step, for example, the fermentation, is carried out in the first side 2 of the hydrophobic membrane 1.

In particular embodiments, the pH of the feed stream 4 has a pH below 6.5. In more particular embodiments the pH of the feed stream 4 is below 6. In more particular embodiments the pH of the feed stream 4 is below 5.5. In more particular embodiments the pH of the feed stream 4 is below 5. In more particular embodiments the pH of the feed stream 4 is in the range from 3 to 6, in particular, from 4 to 5.5, more in particular from 4 to 5, for example 4.2, 4.3, 4.4, 4.5, 4.6, 4.7, 4.8, or 4.9. This is advantageous because when the pH of the feed stream 4 is kept acidic, the C1-C4 carboxylic acids of the feed stream 4 are present in their undissociated form and may more easily cross the hydrophobic membrane 1. On the other side, it is advantageous to keep a pH above 6.5 on the second side of the membrane 3, i.e., the pH of the aqueous microbial culture 5 is kept above 6.5, because then the elongated products will be mostly in the dissociated form (i.e., butyrate, valerate, hexanoate, heptanoate, octanoate), accumulating in the second side 3 instead of diffusing back towards the first side 2, as only undissociated carboxylic acids can cross the hydrophobic membrane 1.

The first aspect further comprises the steps of: bringing an aqueous microbial culture 5 comprising enzymes capable of carrying out carbon chain elongating of organic C1-C4 compounds into contact with the second side 3 of said hydrophobic membrane 1, thereby causing the organic C1-C4 compounds concentration-driven diffusion from said feed stream 4 through said hydrophobic membrane 1 into said aqueous solution culture (5); and subjecting the organic C1-C4 compounds diffused into the aqueous solution comprising enzymes capable of carrying out carbon chain elongating of organic C1-C4 compounds 5 to carbon chain elongation in the presence of an electron donor.

As explained above, the hydrophobic membrane 1 acts as a selective barrier which allows the concentration-driven diffusion of the organic C1-C4 compounds such as ethanol and undissociated SCCA, while at the same time physically separating the aqueous culture comprising enzymes capable of carrying out carbon chain elongating 5 from the feed stream 4. When a solution containing chain-elongating enzymes (pure enzymes or a culture comprising elongating microorganisms) is provided at the second side 3 of the membrane 1, the organic C1-C4 compounds that have diffused through the membrane 1 are used as substrates for carbon chain elongation to produce the elongated compounds, for example, via the reverse β-oxidation (RBO) pathway or the fatty acid biosynthesis (FAB) pathway. This will keep the organic C1-C4 compounds concentration low in the second side 3, further favouring their diffusion from the feed stream 4.

Carbon chain elongation is an enzymatic reaction that enables iterative non-decarboxylative elongation of carbon molecules of varying chain lengths. The carbon chain elongation proceeds with the required presence of an electron donor. The enzymatic carbon chain elongation reactions that may be used in the process of the present disclosure are not limited. In some embodiments, reversed β-oxidation pathway is used for carbon elongation. This pathway has been previously disclosed (EP2271764). However, other pathways may be employed, for example, the fatty biosynthesis pathway (FAB) (Wang et al, 2022).

In one embodiment, the electron donor is selected from the group comprising hydrogen, formate, carbon monoxide, organic C2 -C4 compounds with a degree of reduction higher than 4, or mixtures thereof. The degree of reduction indicates the capacity of a compound to reduce other compounds. It is expressed in number of electrons that are involved in the half reaction of the compound with the compounds in the reference oxidation state. The degree of reduction is the number of electrons involved in this oxidizing half reaction per carbon atom of the compound. Thus, the degree of reduction higher than 4 means that at least 4 electrons are involved in the half reaction of the electron donor.

Suitable electron donors for the process of the first aspect are ethanol, formate, carbon monoxide, methanol, glycerol, galactitol, propanol, lactate, and 1,3-dipropanol acetate. In particular embodiments, the electron donor is selected from hydrogen, ethanol, n-butanol, and mixtures thereof. In more particular embodiments, the electron donor is ethanol. As mentioned above, ethanol diffuses through the hydrophobic, e.g. silicone, membrane 1 at a higher rate than most SCCAs, such as acetic acid, generating high ethanol:SCCA ratios in the second side 3, such high rations being beneficial for chain elongation.

In one embodiment of the first aspect, the chain elongation in the second side 3 is carried out under reductive anaerobic conditions. In some embodiments, the reductive anaerobic conditions in the second side 3 take place at a redox potential (measured against a standard hydrogen electrode) lower than -350 mV, in particular lower than -400 mV, more in particular lower than -450 mV. A particularly suitable range for the redox potential is - 350 to -700 mV, such as -400 to -600 mV, such as -450 to -550 mV.

The elongation may be conveniently carried out by a microbial culture containing elongating microorganisms 5a, as shown in figure 2. Thus, in one embodiment of the first aspect, the aqueous solution 5 comprising enzymes capable of carrying out carbon chain elongation of organic C1-C4 compounds is an aqueous microbial culture 5a comprising microorganisms capable of carrying out chain elongating of organic C1-C4 compounds. The microorganisms in said microbial culture 5a should be suitable for carrying out the carbon chain elongation under reductive anaerobic conditions. Microorganisms suitable for carrying out carbon chain elongation may be found in any sludge from anaerobic digestion process, but also in sewage sludge, ruminal microbial flora, leachates, canal, river or lake sediments, pit mud, stillage, manure, or in the sludge of a reactor fermenting acetate and/or ethanol (or other organic C1-C4 compounds). Accordingly, the microorganisms may originate from an inoculum of such anaerobic sludge or reactor. However, other sources of microorganisms may be used, for example, sources of fermentative bacteria, such as *Clostridium, Caproiciproducens, Eubacterium, Megasphaera* and *Ruminococcaceae.*

In one embodiment, the microbial culture 5a comprises one type of elongating microorganism, for example, *Clostridium.* That is, the bacterial culture 5a may be a pure culture. In another embodiment, the microbial culture 5a comprises more than one elongating microorganisms, for example, two, three, four, five, six, seven, eight, nine or ten different elongating microorganisms. In a particular embodiment, the microbial culture 5a is a defined combination of elongating microorganisms. In another embodiment, the microbial culture 5a originates from an inoculum from sludge or reactor, for example, an inoculum from sewage sludge. In a particular embodiment, the microbial culture 5a comprises microorganisms selected from the group consisting of *Clostridium, Caproiciproducens, Eubacterium, Megasphaera* and *Ruminococcaceae.* In a more particular embodiment, the microbial culture 5a comprises *Clostridium,* more in particular, the microbial culture 5a comprises *Clostridium kluyveri.* In another particular embodiment, the microbial culture 5a comprises *Megasphaera,* more in particular, *Megasphaera hexanoica.*

In some instances, for example, when mixed microorganism cultures are used, it is convenient to inhibit parallel enzymatic reactions, such as the formation of methane. Accordingly, in one embodiment of the process of the first aspect, methane formation is substantially inhibited during carbon chain elongation. According to one embodiment, the methane formation is suppressed or even inhibited by carrying out a heat pre-treatment, freeze-thawing, acidic or alkaline pretreatment, and/or the addition of a methane formation inhibiting agent to the microbial culture 5a. An example of methane formation inhibiting agent is 2-bromo-ethanesulfonic acid.

The process of the present disclosure is particularly advantageous when working with bacterial cultures for carrying out the elongation, since the conditions for optimal growth and activity of the microorganisms in the second side 3 may be selected and tightly controlled, without contamination from the feed stream. This allows, for example, for using pure bacterial cultures, or controlled mixtures of elongating microorganisms.

Regarding temperature conditions, for example, in one embodiment the temperature of the aqueous solution 5 (or the microbial culture 5a) in the second side 3, is in the range from 10 to 55 °C, in particular, the temperature of the aqueous solution 5 in the second side 3 is in the range from 25 to 40 °C, more in particular, the temperature of the aqueous solution 5 in the second side 3 is around 35 °C.

Furthermore, as outlined above, if the pH is the second side 3 is kept above 6.5, the elongated products will be mostly in the dissociated form (i.e., butyrate, valerate, hexanoate, heptanoate, octanoate), accumulating in the second side 3 instead of diffusing back towards the first side 2, as only undissociated carboxylic acids can cross the hydrophobic membrane 1. Therefore, in particular embodiments, the aqueous solution 5 (or the microbial culture 5a) has a pH above 6.5, in particular, a pH in the range from 6.5 to 8, more in particular, a pH around 7.

The process of the first aspect yields mainly C4-C10 carboxylic acids. In one embodiment, the obtained products comprise carboxylic acids selected from C4, C6, C8, and combinations thereof. In another particular embodiment, the obtained products comprise C5-C8 carboxylic acids. In a particular embodiment the obtained products comprise C6-C8, more in particular the obtained products comprise C6 carboxylic acids.

In more particular embodiments the obtained carboxylic acids are in dissociated form, i.e. in de-protonated form. In particular embodiments, the obtained products comprise a dissociated carboxylic acid selected from butyrate, valerate, hexanoate, heptanoate, octanoate, and combinations thereof, more particularly, the obtained products comprise hexanoate.

The products obtained by the process of the first aspect, i.e. C4-C10 carboxylic acids, are further separated from the aqueous solution 5 or microbial culture 5a. The separation may be performed by any method available to the skilled person. For example, the obtained products may be separated by a method selected from the group consisting of extraction, precipitation, flotation, sedimentation, absorption, pertraction, and combinations thereof.

The enzymatic method according to the present invention may be carried out continuously or batch wise, such as fed batch wise. When compared to prior art methods, the present method has the advantage that it can be carried out continuously and, at the same time, adjusting the conditions to optimise carbon chain elongation on the one side and adjusting the conditions for the treatment/pre-treatment of the feed solution, when said treatment is present.

The produced C4 -C10 carboxylic acids may be used as substrates to produce biofuels by a subsequent reaction such as solventogenesis, hydrogenation or ketonic decarboxylation. Thus, a second aspect of the disclosure refers to a process for producing biofuel comprising the step of producing C4-C10 carboxylic acids by a process as defined in the first aspect. All embodiments described above for the first aspect also apply to the second aspect. In one embodiment of this second aspect, the process for producing biofuel further comprises subjecting the C4 -C10 carboxylic acids to covalent coupling. In a particular embodiment, the process for producing biofuel further comprises subjecting the C4 -C10 carboxylic acids to covalent coupling by ketonic decarboxylation. In another embodiment of the second aspect, biofuels are produced by subjecting the C4 -C10 carboxylic acids to catalytic hydrogenation. In another embodiment of the second aspect, biofuels are produced by microorganisms or enzymes *via* reduction of carboxylate to alcohol (also known as solventogenesis).

The process of the invention may be advantageously carried out in a bioreactor. For example, in a configuration of the method, the membrane 1 separates the bioreactor in a first side 2 and a second side 3. Preferably, each side of the bioreactor is equipped with means that enable controlling the conditions inside each side, such as stirrer, recirculation flow control, temperature control and pH control. In a particular configuration the method of the first aspect is carried out in a bioreactor 6 as shown in figure 3. Said bioreactor 6 is, according to the third aspect of the disclosure, configured for performing the process of the first aspect and comprises:
(i) a bioreactor chamber 7, which coincides with the second side 3, filled with an aqueous solution 5 comprising enzymes capable of carrying out chain elongating of organic C1-C4 compounds,
(ii) a polymeric hydrophobic membrane in the form of a tubular element 1a through the lumen of which 8, coinciding with the first side 2, a feed stream 4 comprising organic C1 - C4 compounds can flow, wherein said membrane 1a is submerged in the aqueous solution 5 comprising enzymes capable of carrying out chain elongation, and
(iii) a recirculation system 9 for the feed stream 4.

All the embodiments described above regarding to the hydrophobic membrane 1, the feed stream 4, the aqueous solution 5, and the conditions for elongation also apply to this third aspect. For example, in some embodiments, the tubular element 1 may be a hollow fibre or a plurality of hollow fibres. For example, in particular embodiments, the aqueous solution 5 comprising enzymes capable of carrying out chain elongation is a microbial culture 5a comprising microorganisms capable of carrying out chain elongation.

The bioreactor 7 may further contain an inlet for introducing the aqueous solution 5 comprising enzymes capable of carrying out chain elongating of organic C1-C4 compounds and, when required further components such as extra solution, enzymes, microorganisms or medium, and/or an outlet for extracting the solution containing C4-C10 carboxylic acids. The inlet and the outlet may be the same or different. In most embodiments, the bioreactor 7 is equipped with means that enable controlling the conditions inside, such as stirrer, peristaltic pumps, recirculation system, temperature control and pH control. The bioreactor 7 may further contain a separation device allowing the obtained C4-C10 carboxylic acids to be removed while preferably retaining the remaining compounds (enzymes, microorganisms, substrates, etc) in the bioreactor. In some embodiments, the separation device is a membrane. In a particular embodiment, the membrane is suitable for pertraction (polydimethylsiloxane, polytetrafluotoethylene, polypropilene and similar). In another particular embodiment, the membrane is porous and suitable for ultrafiltration, nanofiltration and/or reverse osmosis (cellulose acetate, polyamide and similar). In another particular embodiment, the membrane is suitable for electrodialysis (ion exchange membranes).

For completeness, the invention is further defined in the following numbered embodiments:
1. A process for the production of C4-C10 carboxylic acid by carbon chain elongation, comprising the steps of:
   (i) providing a hydrophobic membrane 1 with a first side 2 and a second side 3 opposite to said first side 2;
   (ii) bringing a feed stream 4 comprising organic C1-C4 compounds, of which at least one is an electron donor, into contact with said first side 2 of said hydrophobic membrane 1;
   (iii) bringing an aqueous solution 5 comprising enzymes capable of carrying out carbon chain elongating of organic C1-C4 compounds into contact with said second side 3 of said hydrophobic membrane 1, thereby causing the organic C1-C4 compounds concentration-driven diffusion from said feed stream 4 through said hydrophobic membrane 1 to said aqueous solution 5;
   (iv) subjecting the organic C1-C4 compounds diffused into the aqueous solution 5 to enzymatic carbon chain elongation in the presence of the electron donor; and
   (v) separating the formed C4-C10 carboxylic acid from the aqueous solution 5.
2. The process according to embodiment 1, wherein the hydrophobic membrane 1 comprises or consists of dense polymeric material.
3. The process according to any one of embodiments 1-2, wherein the polymeric material is selected from the group consisting of polysiloxanes such as polydimethylsiloxane (silicone), polytetrafluotoethylene, and polypropylene.
4. The process according to any one of embodiments 1-3, wherein the hydrophobic membrane 1 is a tubular element 1a, wherein the first side 2 of the hydrophobic membrane is the lumen of the tubular element, and wherein the feed stream 4 flows through the lumen of the tubular element 1a.
5. The process according to embodiment 3, wherein the tubular element 1a is a hollow fibre, in particular a plurality of hollow fibres.
6. The process according to any one of embodiments 1-5, wherein the step (ii) of bringing said feed stream 4 into contact with the first side 2 of the hydrophobic membrane 1 is carried out by flowing the feed stream 4 through the first side 2 of the hydrophobic membrane 1.
7. The process according to embodiment 6, wherein the feed stream 4 flows through the first side 2 of the hydrophobic membrane 1 at a flow velocity in the range from 1 to 5000 m/h, in particular from 50 to 1000 m/h, more in particular from 100 to 500 m/h.
8. The process according to any one of embodiments 6-7, wherein the feed stream 4 is recirculated after flowing through the first side 2 of the hydrophobic membrane 1.
9. The process according to any one of embodiments 1-8, wherein the hydrophobic membrane 1 has a thickness in the range from 0.01 to 2 mm.
10. The process according to the preceding embodiment, wherein the hydrophobic membrane 1 has a thickness in the range from 0.25 to 1 mm.
11. The process according to any one of embodiments 1-10, wherein the feed stream 4 comprises ethanol and, optionally, other organic C1-C4 compounds selected from alcohols, undissociated carboxylic acids, and mixtures thereof.
12. The process according to the preceding embodiment, wherein the feed stream comprises ethanol in a concentration of at least 0.01 M, in particular at least 0.1 M, more in particular from 0.1 to 1M.
13. The process according to any one of embodiments 11-12, wherein the C1-C4 carboxylic acids comprise acetic acid, propionic acid, butyric acid, or mixtures thereof.
14. The process according to any one of embodiments 1-13, wherein the feed stream 4 comprises ethanol and undissociated acetic acid.
15. The process according to any one of embodiments 1-14, wherein the feed stream 4 comprises or consists of carbon containing waste or by-products.
16. The process according to the preceding embodiment wherein the carbon containing waste or by-products is organic waste or by-products, in particular selected from wastewater, sewage, distillery/liquor making wastewater, municipal organic waste, agroindustrial waste, wine lees, winery wastewater, beer factory wastewater, whey, lignocellulosics, stillage, molasses, sugars.
17. The process according to the preceding embodiment wherein the organic waste or by-product is fermented.
18. The process according to any one of embodiments 1-17, wherein the feed stream 4 comprises or consists of fermented biomass or fractions thereof.
19. The process according to embodiment 15, wherein the carbon-containing substrates are produced from CO₂ and CO from industrial flue gas.
20. The process according to any one of embodiments 1-19, wherein the feed stream 4 has a pH below 6, in particular a pH from 3 to 5.
21. The process according to any one of embodiments 1-20, wherein in step (iv) the electron donor is ethanol which has diffused from the feed stream 4 into the aqueous culture 5 through the hydrophobic membrane 1.
22. The process according to the preceding embodiment, wherein the ethanol:acetic acid ratio in the second side 3 is in the range from 3:1 to 10:1, in particular from 5:1 to 6:1.
23. The process according to any one of embodiments 1-22, wherein the carbon chain elongation is carried out by microorganisms in an aqueous microbial culture 5a, preferably under reductive anaerobic conditions at a redox potential lower than -350 mV, in particular lower than - 400 mV, more in particular lower than -450 mV.
24. The process according to the preceding embodiment, wherein the microorganisms are fermentative bacteria.
25. The process according to the preceding embodiment, wherein the microorganisms are selected from the group consisting of *Clostridium, Caproiciproducens, Eubacterium, Megasphaera, Ruminococcaceae,* and combinations thereof.
26. The process according to the preceding embodiment, wherein the microorganisms comprise at least one selected from *Clostridium kluyveri, Megasphaera hexanoica,* and combinations thereof.
27. The process according to any one of embodiments 1-26, wherein the microorganisms originate from an inoculum from anaerobic sludge.
28. The process according to any one of embodiments 1-27, wherein the aqueous solution 5 has a pH above 6.5, in particular, a pH in the range from 6.5 to 8, more in particular, a pH in the range from 6.5 to 7.5.
29. The process according to any one of embodiments 1-28, wherein the temperature of the aqueous solution 5 is in the range from 10 to 55 °C, in particular, in the range from 25 to 40 °C, more in particular, around 35 °C.
30. The process according to any one of embodiments 1-29, wherein the obtained products comprise C6-C10 carboxylic acids.
31. The process according to any one of embodiments 1-30, wherein the obtained products comprise C6-C10 carboxylic acids in dissociated form.
32. The process according to any one of embodiments 1-31, wherein the obtained products comprise a dissociated carboxylic acid selected from valerate, hexanoate, heptanoate, octanoate, and combinations thereof.
33. The process according to any one of embodiments 1-32, wherein the obtained products are separated by a separation method selected from extraction, precipitation, flotation, sedimentation, absorption, and combinations thereof.
34. The process according to any one of embodiments 1-33, wherein during the enzymatic carbon chain elongation step (iv) methane formation is substantially inhibited.
35. The process according to the preceding embodiment, wherein methane formation is inhibited by adding a methane formation inhibiting agent in the second side 3, in particular, by adding 2-bromo-ethanesulfonic acid.
36. The process according to any one of embodiments 1-35, further comprising a step of pre-treating of the feed stream 4.
37. The process according to the preceding embodiment, wherein the pre-treatment is selected from the group consisting of fermentation, electrofermentation, milling, grinding, pressing heating, acid-base treatment, oxidation, enzymatic hydrolysis, and combinations thereof.
38. The process according to any one of embodiments 36-37, wherein the pre-treatment is carried out in a separate equipment.
39. The process according to any one of embodiments 1-38, wherein the aqueous solution 5 is contained in a bioreactor.
40. The process according to the preceding embodiment, wherein the membrane 1 is a tubular element 1a submerged in the bioreactor.
41. The process according to the preceding embodiment, wherein the tubular element 1a is a hollow fibre, in particular a plurality of hollow fibres.
42. The process according to any one of embodiments 39-41, wherein the bioreactor is equipped with magnetic stirrer, recirculation, temperature control and pH control.
43. A process for the production of biofuel comprising producing C4 -C10 carboxylic acids by a process as defined in any one of embodiments 1-42.
44. The process for the production of biofuel according to the preceding embodiment, further comprising subjecting the C4 -C10 carboxylic acids to covalent coupling, hydrogenation or solventogenesis.
45. A bioreactor 6 configured for performing the process according to any of the preceding claims, said bioreactor 6 comprising:
   (i) a bioreactor chamber 7 filled with an aqueous solution 5 comprising enzymes capable of carrying out chain elongating of organic C1-C4 compounds,
   (ii) a polymeric hydrophobic membrane in the form of a tubular element 1a through the inside of which 8 a feed stream 4 comprising organic C1 -C4 compounds can flow, wherein said membrane 1a is submerged in the aqueous solution 5 comprising enzymes capable of carrying out chain elongation, and
   (iii) a recirculation system 9 for the feed stream 4.

Throughout the description and claims the word "comprise" and variations of the word, are not intended to exclude other technical features, additives, components, or steps.

Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Examples

A system according to the present invention has been set up at laboratory scale using synthetic solutions. The experimental set-up consisted of bioreactor 7 containing a cylindric fermenter of 2 L volume, in which a silicone tubing coil of 6.67 m length was submerged (see SIL-HEX picture in Figure 4). The commercial silicone tubular membrane had an internal and external diameter of 2 and 3 mm, respectively, resulting in a 0.5 mm wall thickness. The fermenter was equipped with magnetic stirrer, recirculation (by peristaltic pump), temperature control (water bath) and pH control (consisting of a pH controller with relay connected to a peristaltic pump to dose 3 M NaOH when required). A third peristaltic pump is used to recirculate the ethanol-containing medium inside the silicone tubing.

The proof-of-concept has been demonstrated by preparing 1 L of a solution (i.e., feeding solution) containing 1 M ethanol and 1 M acetic acid, at pH 4.5. Such a solution was recirculated at 21 mL/min inside the silicone tubing coil located in the fermenter, resulting in a flow velocity of 100 m/h. The fermenter was filled with 1.4 L of an inorganic medium with the following composition (in g/L): KH₂PO₄ (0.2), NaCl (1.6), NH₄Cl (2.0); MgCl x 6H₂O (0.4), KCI (0.2), CaCl₂ x 2H₂O (0.04), and MES buffer (3.9). It also included a trace metal solution with the following composition (in mg/L): Nitrilotriacetic acid (20.0), MnSO₄ x H₂O (10.0), Fe(SO₄)₂(NH₄)₂ x 6H₂O (8.0), CoCl₂ x 6H₂O (2.0), ZnSO₄ x 7H₂O (0.02),CuCl₂ x 2H₂O (0.2), NiCl x 2H₂O (0.2), Na₂MoO₄ x 2H₂O (0.2). Moreover, it contained a mix vitamin solution with the following composition (mg/L): Biotin (20.0), Folic acid (20.0), Pyridoxine hydrochloride (100.0), Thiamine hydrochloride (50.0), Riboflavin (50.0), Nicotinic acid (50.0), DL-calcium pantothenate (50.0), Vitamin B12 (1.0). The fermenter was inoculated at a ratio of 5% v/v with a mixed microbial population of chain elongating microorganisms, dominated by *Megasphaera* sp., originating from swine manure and previously cultivated in our lab. The fermenter medium was mixed by both magnetic stirring and recirculation (170 mL/min) and maintained under optimal conditions for chain elongation (pH 7.0 ± 0.2 and at 35 ± 1 °C). It was sparged with N₂ before the experiment to strip out O₂.

Results showed that both acetic acid and ethanol diffused towards the fermenter, with a preference for ethanol, obtaining a ethanol:acetic acid ratio of around 5 after two days operation. From day 3, ethanol concentration declined and elongated products (mainly hexanoate) appeared confirming the onset of chain elongation pathways (Figure 5). Hexanoate was produced at a highest rate of 3.2 g/(L.d) and accumulated to 15 g/L after three weeks operation. Its production selectivity was >90% (on a carbon basis), with small concentration of butyrate. Some unreacted ethanol remained in the fermenter, suggesting that its diffusion was exceeding the metabolic capability of chain elongating microorganisms. However, the ethanol dosing can be simply fine-tuned (e.g., by reducing the length of the tubing coil) to avoid its accumulation, resulting in a relatively pure (>90%) hexanoate product. None of those products were detected in the feeding solution, confirming that the silicone membrane prevents the diffusion of dissociated carboxylic acids. Hence, the proof of concept was successfully demonstrated in the lab.

### Citation List

- EP2271764
- He et al, 2021. "Medium chain fatty acids production from simple substrate and waste activated sludge with ethanol as the electron donor". Chemosphere 283, 131278. DOI: 10.1016/j.chemosphere.2021.131278
- Huo et al, 2022. "Strategy of electron acceptors for ethanol-driven chain elongation from kitchen waste". Science of The Total Environment 846, 157492. DOI: 10.1016/j.scitotenv.2022.157492.
- Wang et al, 2022. "Biological production of medium-chain carboxylates through chain elongation: An overview". Biotechnology Advances 55,107882. DOI: 10.1016/j.biotechadv.2021.107882.

## Claims

1. A process for the production of C4-C10 carboxylic acid by carbon chain elongation, comprising the steps of:
(i) providing a hydrophobic membrane (1) with a first side (2) and a second side (3) opposite to said first side (2);
(ii) bringing a feed stream (4) comprising organic C1-C4 compounds, of which at least one is an electron donor, into contact with said first side (2) of said hydrophobic membrane (1);
(iii) bringing an aqueous solution (5) comprising enzymes capable of carrying out carbon chain elongating of organic C1-C4 compounds into contact with said second side (3) of said hydrophobic membrane (1), thereby causing the organic C1-C4 compounds concentration-driven diffusion from said feed stream (4) through said hydrophobic membrane (1) to said aqueous solution (5);
(iv) subjecting the organic C1-C4 compounds diffused into the aqueous solution (5) to enzymatic carbon chain elongation in the presence of the electron donor; and
(v) separating the formed C4-C10 carboxylic acid from the aqueous solution (5).

2. The process according to claim 1, wherein the hydrophobic membrane (1) comprises or consists of dense polymeric material, in particular the dense polymeric material is selected from the group consisting of polysiloxanes such as polydimethylsiloxane (silicone), polytetrafluotoethylene, and polypropylene.

3. The process according to any one of claim 1-2, wherein the hydrophobic membrane (1) is a tubular element (1a), wherein the first side (2) of the hydrophobic membrane is the lumen of the tubular element, and wherein the feed stream (4) flows through the lumen of the tubular element (1a).

4. The process according to any one of claims 1-2, wherein the hydrophobic membrane (1) has a thickness in the range of 0.01 to 2 mm.

5. The process according to any one of claims 1-4, wherein the feed stream (4) comprises ethanol and, optionally, acetic acid.

6. The process according to the preceding claim, wherein the feed stream (4) comprises at least 0.01 M, in particular at least 0.1 M, more in particular from 0.1 to 1M, of ethanol.

7. The process according to any one of claims 1-6, wherein the feed stream (4) comprises carbon containing waste or by-products, in particular the feed stream (4) is selected from wastewater, sewage, distillery/liquor making wastewater, fermented municipal organic waste, agroindustrial waste, wine lees, winery wastewater, beer factory wastewater, whey fermentate, fermented lignocellulosics, stillage, molasses, sugars, and soluble carbon containing products obtained from CO₂ and CO from industrial flue gas.

8. The process according to any one of claims 1-7, wherein the feed stream (4) is fermented biomass.

9. The process according to any one of claims 1-8, wherein in step (iv) the electron donor is ethanol which has diffused from the feed stream (4) into the aqueous culture (5) through the hydrophobic membrane (1).

10. The process according to any one of claims 1-9, wherein the aqueous culture (5) is a microbial culture comprising microorganisms capable of carrying out carbon chain elongating of organic C1-C4 compounds, in particular, the microbial culture comprises microorganisms selected from the group consisting of *Clostridium, Caproiciproducens, Eubacterium, Megasphaera, Ruminococcaceae,* and combinations thereof.

11. The process according to any one of claims 1-10, wherein the feed stream (4) has a pH below 6, in particular a pH from 3 to 5, and/or the aqueous solution (5) has a pH above 6.5, in particular, a pH in the range from 6.5 to 8.

12. The process according to any one of claims 1-11, wherein the obtained products comprise carboxylic acids selected from valerate, hexanoate, heptanoate, octanoate, and combinations thereof.

13. The process according to any one of claims 1-12, wherein step (v) comprises a separation method selected from extraction, precipitation, flotation, sedimentation, absorption, and combinations thereof.

14. A process for the production of biofuel comprising producing C4-C10 carboxylic acids by a process as defined in any one of the preceding claims and, optionally, subjecting the C4-C10 carboxylic acids to covalent coupling, hydrogenation or solventogenesis.

15. A bioreactor (6) configured for performing the process according to any of the preceding claims, said bioreactor (6) comprising:
(i) a bioreactor chamber (7) filled with an aqueous solution (5) comprising enzymes capable of carrying out chain elongating of organic C1-C4 compounds,
(ii) a polymeric hydrophobic membrane in the form of a tubular element 1a through the inside of which (8) a feed stream (4) comprising organic C1 -C4 compounds can flow, wherein said membrane (1a) is submerged in the aqueous solution (5) comprising enzymes capable of carrying out chain elongation, and
(iii) a recirculation system (9) for the feed stream (4).
